# EUROPEAN PATENT SPECIFICATION

(11) **EP 0 436 547 B1**
(45) Date of publication and mention of the grant of the patent: **30.11.1994**
(21) Application number: 89906981.9
(22) Date of filing: 10.05.1989
(51) Int. Cl.: C12Q 1/68

(54) **PROCESS FOR RAPID NUCLEIC ACID DETECTION**
VERFAHREN ZUM SCHNELLEN NACHWEIS VON NUKLEINSÄUREN
PROCEDE DE DETECTION RAPIDE D'ACIDE NUCLEIQUE

(30) Priority: 10.05.1988 US 192090
(43) Date of publication of application: 17.07.1991
(73) Proprietor: E.I. DU PONT DE NEMOURS AND COMPANY, Wilmington Delaware 19898 (US)
(72) Inventor: ADLER, Karl, E., Newburyport, MA 01950 (US); GREENE, Richard, Alfred, Westford, MA 01886 (US); KASILA, Patricia, Ann, Windham, NH 03087 (US); MILLER, Jeffrey, Allan, Derry, NH 03038 (US)
(74) Representative: Cresswell, Thomas Anthony
(86) International application number: US8901996
(87) International publication number: WO8910979

(56) References cited:
- EP-A- 139 489
- EP-A- 192 168
- EP-A- 200 113
- EP-A- 0 229 701
- WO-A-85/05451
- WO-A-86/03782
- WO-A-86/05815
- DE-A- 3 546 312
- GB-A- 1 420 916
- US-A- 4 512 896
- US-A- 4 563 419
- US-A- 4 683 195
- US-A- 4 683 202
- US-A- 4 708 932
- JOURNAL OF AGRICULTURAL SCIENCE, vol. 59, no. 3, 1987, University of Helsinki (FI); R. KARJALAINEN et al., abstract#
- JOURNAL OF VIROLOGY, vol. 61, no. 5, 1987, American Society of Microbiology, Washington, DC (US); S. KWOK et al., pp. 1690-1694#
- NATURE, vol.331, February 1988, MacMillan Magazines LTD., London (GB); H.A.ERLICH et al., pp. 461-462#

## Description

### FIELD OF INVENTION

This invention relates to nucleic acid hybridization for the detection of nucleic acid sequences and more specifically to a process of combining amplification of target nucleotide sequences with solution sandwich hybridization of the amplified target material.

### BACKGROUND OF THE INVENTION

The development of practical nucleic acid hybridization methods which can be used for detecting nucleic acid sequences of interest has been limited by several factors. These include lack of sensitivity, complexity of procedure, and the desire to convert from radiometric to nonradiometric detection methods. A variety of methods have been investigated for the purpose of increasing the sensitivity of nonradiometric procedures. In one general approach, improvements in the total assay procedure have been examined, with concomitant effects on the issues of complexity and nonradiometric detection. In another approach, methods which increase the amount of nucleic acid to be detected by such assays have been pursued.

U.S. Patent 4,358,535, issued to Falkow, describes a method of culturing cells to increase their number and thus the amount of nucleic acid of the organism suspected to be present, depositing the sample onto fixed support, and then contacting the sample with a labeled probe, followed by washing the support and detecting the label. One drawback to this method is that without culturing the organism first, the assay does not have adequate sensitivity. Adding a culture step, however, is time consuming and not always successful. Maniatis et al., Molecular Cloning: A Laboratory Manual, Cold Spring Harbor Laboratory, pp.390-401 (1982), describe a method in which a nucleic acid of interest is amplified by cloning it into an appropriate host system. Then, when the host organism replicates in culture, the nucleic acid of interest is also replicated. This method also suffers from the requirement to perform a culture step and thus provides for a procedure that is time consuming and complicated.

An alternative approach to increasing the quantity of nucleic acids of organisms has been described in U.S. patents 4,683,202 and 4,683,195. These patents disclose "a process for amplification and detection of any target nucleic acid sequence contained in a nucleic acid or mixture thereof". This process employs an in vitro cycling mechanism which doubles the nucleic acid sequence to be amplified after each cycle is complete. This is carried out by separating the complementary strands of the nucleic acid sequence to be amplified, contacting these strands with excess oligonucleotide primers and extending the primers by enzymatic treatment to form primer extension products that are complementary to the nucleic acid annealed with each primer. The process is then repeated as many times as is necessary. An advantage of this method is that it can rapidly produce large quantities of a small portion of the sequence of the nucleic acid of an organism of interest. A disadvantage of this method is that the detection of the nucleic acids produced. using a direct assay method, is complicated in that the amplification process can produce nucleic acid sequences which are not faithful copies of the original nucleic acid which was to be copied. These erroneous nucleic acid sequences can provide false positives in the assay which increase the background noise and thus decrease the sensitivity of the entire method.

Numerous DNA probe assays have been described in the past for the detection of nucleic acids of interest. Falkow's method (above) first renders the target nucleic acid single-stranded and then immobilizes it onto a solid support. A labeled probe which is complementary to the target nucleic acid is then brought into contact with the solid support. Any excess probe is washed away and the presence of the label in the resulting hybrid is determined. A disadvantage of this method is that it is time consuming and cumbersome. The assay steps, i.e., hybridization and washing steps are carried out in a sealed pouch which contains the membrane (solid support) as well as the buffer solution.

Hill et al., WO 86/05815, describe a variation of the above assay format employing nitrocellulose coated magnetic particles to which the target DNA is affixed, followed by direct hybridization with a biotinylated probe and detection using a streptavidin-conjugated reporter.

Dunn et al.. Cell, Vol. 12, 23-36 (1977), describe a different hybridization format which employs a two-step sandwich assay method employing polynucleotide probes in which the target nucleic acid is mixed with a solution containing a first or capture probe which has been affixed to a solid support. After a period of time, the support is washed and a second or reporter (labeled) probe, also complementary to the target nucleic acid but not to the capture probe, is added and allowed to hybridize with the capture probe - target nucleic acid complex. After washing to remove any unhybridized reporter probe, the presence of the reporter probe, hybridized to the target nucleic acid, is detected.

Ranki et al. U.S. patent 4,563,419, disclose a one-step sandwich hybridization assay in which a capture probe affixed to a solid support and a reporter probe are added simultaneously to the target nucleic acid followed by the removal of any unhybridized reporter probe. This assay is an improvement over the two-step sandwich hybridization method because it simplifies the procedure by eliminating one washing step and increases the sensitivity.

Several variations of the one-step assay have been described, see, for example, EPA 0 139 489, EPA 0 154 505, W086/03782, and EPA 0 200 113. It is to be recognized that all of these employ an assay procedure in which the first or capture probe is immobilized onto a solid support prior to hybridization.

A further variation has been described in German Preliminary Published Application 3,546,312 A1. This method, like that described by Ranki et a1., employs a capture probe and a reporter probe which hybridize to distinct portions of the target nucleic acid. The target nucleic acid is contacted in solution by the two probes. The first, or capture probe, contains a binding component, such as biotin, that is capable of binding with a receptor component, such as streptavidin, which has been affixed to a solid support. After formation of the capture probe - target nucleic acid - reporter probe complex, a streptavidin-modified solid support is added. Any unhybridized reporter probe is washed away followed by the detection of the label incorporated into the complex bound to the solid support. An advantage of this technique over that disclosed by Ranki et al is that the hybridization, which takes place in solution, is favored kinetically. Some disadvantages are that the length of the target nucleic acid affects the overall efficiency of the reaction which decreases with increasing target nucleic acid length. Also, sandwich nucleic acid probe assays, whether heterogeneous two-step or one-step, or utilizing solution hybridization, are not as sensitive as the direct assay method.

A method of identifying human virus sequences by using in vitro enzymatic amplification and oligomer cleavage detection is disclosed in J. Virology, 61, (5), 1690-1694 (1987).

A method of carrying out PCR is disclosed in Nature, 331, 461-462 (1988).

EP-A-192168 discloses a solution hybridisation coupled with a hybrid separation system.

GB-A-1420916 discloses a reaction cell for the performance of radioimmunoassay determinations having supported within it a matrix pad of absorbent material capable of retaining the necessary reagents for the reaction.

### DISCLOSURE OF THE INVENTION

The nucleic acid probe assay of this invention for the detection and/or measurement of a preselected nucleic acid sequence in a sample suspected of including a nucleic acid containing said preselected sequence comprises the steps of:
(A) rendering the target nucleic acid single-stranded;
(B) amplifying at least one specific nucleic acid sequence contained within the preselected nucleic acid sequence by
   (1) treating the strands with two oligonucleotide primers, for each different specific sequence being amplified, under conditions such that for each different sequence being amplified an extension product of each primer is synthesized which is complementary to each nucleic acid strand, wherein said primers are selected so as to be sufficiently complementary to the different strands of each specific sequence to hybridize therewith such that the extension product synthesized from one primer, when it is separated from its complement, can serve as a template for synthesis of the extension product of the other primer;
   (2) separating the primer extension products from the templates on which they were synthesized to produce single-stranded molecules; and
   (3) treating the single-stranded molecules generated from step (2) with the primers of step (1) under conditions that a primer extension product is synthesized using each of the single strands produced in step (2) as a template:
   (4) repeating steps (2) and (3) to produce sufficient primer extension product for detection and/or measurement:
(C) rendering the product of step (B)(4) single-stranded;
(D) contacting the product of step (C) with capture and reporter probes in solution wherein said capture probe is complementary to a portion of a primer extension product not including the nucleic acid sequences defined by both primers; wherein said reporter probe is complementary to a portion of a primer extension product not including the nucleic acid sequences defined by the capture probe and by both primers; and wherein the capture and reporter probes are complementary to the same nucleic acid strand;
(E) immobilizing the sandwich product of step (D) by contacting it with a solid support having an anchor receptor group on its surface capable of interacting with the anchor group of the capture probe;
(F) removing any unhybridized reporter probe; and
(G) detecting and/or measuring the reporter group immobilized on the solid support.

### DETAILED DESCRIPTION OF THE INVENTION

The nucleic acid probe assay of this invention comprises the following overall process for the detection of target nucleic acids of a preselected sequence:
a) Using the polymerase chain reaction (PCR) nucleic acid amplification method described in U.S. 4.683,202, specific nucleic acid sequences are amplified by annealing the denatured target nucleic acid present in the sample with primers specific for the target and forming extension products. Each extension product formed is complementary to a portion of the preselected nucleic acid sequence contained within the target nucleic acid and becomes a template for further primer binding. This process is then repeated as necessary in order to produce the desired amount of primer extension product for detection and/or measurement.
b) The resulting nucleic acid is rendered single-stranded by known methods, such as treatment with heat, chaotropic agents, or by raising or lowering the pH. The single-stranded nucleic acid so produced is then contacted, simultaneously or sequentially, with buffered solutions containing nucleic acid capture and reporter probes, respectively. The first probe, designated the capture probe, is an oligonucleotide (nucleic acid) sequence, which is complementary to a portion of the target nucleic acid, but not to the primer utilized in the amplification step, to which is attached a functional group designated as the anchor. The second probe, designated the reporter probe, is an oligonucleotide (nucleic acid) sequence, which is complementary to a portion of the target nucleic acid sequence distinct from the sequence complementary to the capture probe and from that of the primers, to which is attached a reporter group. The capture probe and the reporter probe must be complementary to the same nucleic acid strand. The result is the formation of a sandwich containing the target nucleic acid, the capture probe, and the reporter probe.
c) The nucleic acid sandwich is then captured from solution by interaction of the anchor group on the capture probe and an anchor receptor which is attached to a solid surface. The captured sandwich is washed with appropriate buffers to remove unhybridized reporter probe. The presence and quantity of the reporter group on the support is then detected and/or measured and is proportional to the amount of amplified target nucleic acid. The amount of amplified target nucleic acid present, in turn, is proportional to the unamplified target nucleic acid originally present in the sample.

The term "PCR" as used herein in referring to the process of amplifying target nucleic acid sequences employing primer oligonucleotides to produce by enzymatic means a greatly increased number of copies of a small portion of the target nucleic acid is described in U.S. patent 4,683,202.

The term capture probe as used herein refers to an oligonucleotide which is complementary to a portion of a preselected sequence of the target nucleic acid and which has attached to it a functional group referred to as an anchor. The capture probe cannot be complementary to either primer or to those portions of a primer extension product whose nucleic acid sequences are defined by the primers. The capture probe is an oligonucleotide with an attached anchor group. Anchor group attachment can be achieved, for example, by modifying an oligonucleotide at a predetermined nucleotide base such that a linker arm of at least three atoms length is added. This linker arm is capable of being attached to the anchor, which is complementary to an anchor receptor. The anchor receptor is positioned on the surface of a solid support and is used to immobilize the capture probe.

The term reporter probe as used herein refers to an oligonucleotide which is complementary to a portion of a preselected sequence of the target nucleic acid which sequence is distinct from the portion of the target nucleic acid which is complementary to the capture probe. The reporter probe cannot be complementary to either primer nor to those portions of a primer extension product whose nucleic acid sequences are defined by the primers nor to the capture probe. A reporter group is covalently attached to the oligonucleotide to form the reporter probe. This attachment can be through a linker arm as described above.

The assay of this invention requires that both the capture probe and the reporter probe be complementary to the same nucleic acid strand.

Useful reporter groups for the reporter probes of this invention include any moiety detectable subsequent to hybridization and immobilization events such as enzymes, fluorophores, chemiluminescent compounds, chromogens, and chromophores. Among enzymes are included alkaline phosphatase, horseradish peroxidase, and luciferase.

Useful anchor groups for the capture probes of this invention include any moiety capable of interacting with anchor receptor groups attached to the surface of said support to permit immobilization of the sandwich formed in the hybridization process. For example, anchors can be members of any specific immune or non-immune binding pair such as antigen-antibody, protein A-IgG, and biotin-avidin or streptavidin.

Solution sandwich hybridization as used herein refers to a process of contacting the target nucleic acid simultaneously or sequentially, with the capture and reporter oligonucleotide probes. It is preferred that there be a simultaneous addition of a solution of both probes to a solution of the target nucleic acid.

The PCR target amplification reaction requires approximately 20 to 30 repeat cycles in order to produce a sufficient quantity of the amplified target nucleic acid for further hybridization. Denaturation of the amplified nucleic acid can be accomplished by treatment with alkali. acid, chaotropic agents, or heat, although the preferred method is to place the amplified target nucleic acid in a boiling water bath for at least 10 minutes followed by a chilled water bath (4°C) for at least two minutes.

Solution hybridization can be accomplished by contacting the single stranded target nucleic acid in solution with both capture and reporter probes, dissolved in an appropriate buffer, for a period of from 1 to 30 minutes, preferably for 10 minutes. Preferably, both probes are used in excess. The length of the capture probe is determined by the ease of its synthesis, by the desired reaction kinetics, and by the identity of the reporter probe and the primers, and preferably is an oligonucleotide of approximately 20 to 30 nucleotide bases. The length of the reporter probe is determined by the case of its synthesis, by the desired reaction kinetics, and by the identity of the capture probe and the primers, and preferably is an oligonucleotide of approximately 20 to 30 nucleotide bases.

The capture probe - target nucleic acid - reporter probe sandwich so formed is then contacted with a solid support having an anchor receptor on its surface capable of forming a stable attachment such as a complex with the sandwich through the anchor group of the capture probe. A variety of solid supports and anchor receptors can be utilized. Among solid supports are included magnetic particles such as the chromium dioxide particles disclosed by Lau et al., U.S. Patent 4,661,408, microtiter plates, or membranes. A preferred membrane incorporated in a device useful in carrying out the assay of this invention is described in US-A-5 137 804.

This device is an improved assay device for detecting or quantitating the presence or absence of a substance in a sample suspected or known to contain said substance, said device having multiple layers comprising: (a) a permeable membrane having a capture reagent attached thereto, (b) an intermediate layer, and (c) an absorbent layer, wherein layer (b) is in direct communication with layers (a) and (c), the improvement wherein layer (b) is a selectively permeable membrane and has at least one hole therethrough, the hole is directly below the capture reagent, and the area of said hole or the combined area of a plurality of holes is less than the area covered by the capture reagent. The term "selectively permeable" refers to materials which do not permit the substantial passage of aqueous solutions therethrough whether or not they permit the passage of other liquids such as organic solvents, if present.

Materials which can be used for the permeable layer, on which the capture reagent is spotted, include various natural or synthetic materials, which may be individual materials or combinations of materials, which may be organic, inorganic or combinations thereof, the permeable layer must be bibulous, i.e., it allows the flow of aqueous solutions therethrough without substantially impeding the movement of solutes employed in the assay. The material selected must also be one to which the capture reagent can be attached to a localized area of the device, either covalently or non-covalently. directly or indirectly as is discussed below. Exemplary materials which may find use are polysaccharides, e.g., cellulosic materials. such as paper, cellulose acetate, nitrocellulose, and backed nitrocellulose: inorganic materials, such as silica, deactivated alumina, diatomaceous earth, MgSO₄ or other inorganic finely divided material substantially uniformly dispersed in a porous polymer matrix, with polymers such as vinyl chloride, vinyl chloride-propylene copolymer, and vinyl chloride-vinyl acetate copolymer; cloth, both naturally occurring, e.g., cotton and synthetic, e.g., nylon cloth; porous gels, e.g., silica gel, agarose, dextran, and gelatin; polymeric films, e.g., polyacrylamide or the like.

There can also be mentioned, in particular. membranes to which proteins can be covalently attached. The list includes the following which can be purchased commercially: microporous affinity membranes with a pore size in the range of about 0.5 to about 5 micrometers, membranes with a chemically preactivated surface which offer a high density of covalent binding sites that immobilize proteins on contact, and chemically activated hydrophilic microporous membranes wherein the base membrane is hydrophilic polyvinylidene fluoride, chemically derivatized to allow protein immobilization through epsilon amino groups of lysine or arginine in the pH range of 7 to 9. For a sensitive assay, the choice of membrane depends primarily upon the ability to prevent nonspecific binding on the membrane by blocking it. This in turn depends upon the reagents selected, the blocking agent, and the membrane itself. The preferred membrane in practicing the invention is a chemically activated hydrophilic microporous membrane.

The selectively permeable layer of this multi-layer device prevents the flow of aqueous solutions under the assay conditions. This resistance to flow is important because it increases the amount of time the capture reagent contacts the other reagents used in the assay, thus improving subsequent steps in building the sandwich and assaying the enzyme, as well as the overall sensitivity of the assay. A wide variety of compositions of known flow characteristics can be used for the selectively permeable layer which includes polyethylene, polyethylene-backed polytetrafluoroethylene and fibrous-porous polytetrafluoroethylene. A fibrous-porous polytetrafluoroethylene membrane is the preferred material for the selectively permeable layer in practicing the instant invention. The fibrous-porous nature of the membrane appears to encourage radial flow along the surface so that liquid flows to the center where the holes are located in the selectively permeable layer. During the assay, no aqueous reagents pass through the pores in the fibrous-porous polytetrafluoroethylene membrane, which is selectively permeable with respect to the aqueous assay reagents.

The absorbent layer serves as a repository for excess reagent solutions. Consequently, all the reactants needed to produce color are present in the absorbent layer. Since development of color in the absorbent layer is not desirable, a variety of reagents can be added to the absorbent layer to reduce production of background color in the absorbent layer. According to one embodiment of the present invention, surprisingly and unexpectedly it has been found that when an enzyme-based detection system is used, color development in the absorbent layer can be minimized by saturation with a solution containing an enzyme inhibitor specific for the enzyme selected to detect.

To remove non-hybridized reporter probe, the immobilized sandwich can then be washed several times, for example, in the temperature range of 25°C - 37°C, for approximately 5 to 10 minutes per wash cycle.

A variety of known detection methods can be utilized in the assay of this invention depending on the type of the reporter groups present on the reporter probes. In general, when the reporter group is an enzyme, a substrate or substrates specific for that enzyme is used along with all other necessary reagents. Color formation can then be detected and/or measured photometrically. When the reporter group is the enzyme alkaline phosphatase, then the preferred substrates are 4-methylumbelliferyl phosphate or p-nitrophenyl phosphate when the solid support is a magnetic particle or a microtiter plate, and the combination of nitro-blue tetrazolium (NBT) and 5-bromo-4-chloro-3-indolyl phosphate (BCIP) when the solid support is the modified membrane described above.

It has been found unexpectedly that when the assay of this invention is carried out, the sensitivity of such an assay is significantly improved over previous DNA probe assay processes. When PCR methodology was combined with a direct hybridization assay ("dot blot"), 1,000 copies of HIV I DNA could be detected using chromogenic detection. Since such a direct assay is known to be more sensitive than the solution sandwich assay, it was particularly surprising to find that the solution sandwich assay format on a membrane device which is one embodiment of the present application, when combined with the PCR methodology in the assay of this invention, again using chromogenic detection, detected 100 copies of HIV I DNA, a 10-fold improvement. When the assay of this invention utilized fluorescent detection, irrespective of the type of solid support, there was also found an increased detection level to 100 copies of HIV I DNA. Furthermore, fluorescent detection methodology cannot be utilized with the direct (dot blot) assay, which is thus limited to a less sensitive detection limit.

To appreciate the sensitivity of the nucleic acid probe assay of this invention, it has to be noted that the absolute sensitivity, as indicated by using only 5% of the amplified target DNA produced by the PCR portion of the assay process, was equal to 5 copies of unamplified starting HIV I DNA. Such results were obtained using the membrane device of this invention, with fluorescent detection combined with the streptavidin-coated microtiter plate or streptavidin coated chromium dioxide particles. This level of sensitivity represents a hitherto unachievable goal with this type of assay.

The Examples below exemplify the invention.

### EXAMPLE I

### Detection of HIV I

### A. Amplification of Target Nucleic Acid by PCR

The procedure as described in U.S. Patent 4,683,202 and in a product bulletin for GeneAmp DNA Amplification Reagent Kit (#N801-0043) was followed utilizing the following specific conditions and reagents. A 103-nucleotide base sequence located within the GAG p17 region of HIV I, incorporated into a plasmid (the plasmid incorporating most of the HIV I genome is designated pBH10-R3), was amplified using primers And B as shown below:

5'-TGGGCAAGCAGGGAGCTAGG
Primer A
5'-TCTGAAGGGATGGTTGTAGC
Primer B

Aliquots of serial dilutions (1x10⁺⁷, 1x10⁺⁶, 1x10⁺⁵, 1x10⁺⁴, 1x10⁺³, 1x10⁺², 1x10⁺¹, and zero copies) of plasmid pBH10-R3 were amplified using PCR. Each aliquot was combined with a buffer which was 200 µM in each of dATP, dTTP, dCTP, and dGTP, 1.0 µM in each of Primer A and Primer B, and contained 1 µg of human placental DNA/reaction and 2.5 units of TAQ polymerase, in a total reaction volume of 100 µl.

Each reaction mixture was then temperature cycled as described in the product bulletin thirty (30) times.

This process resulted in the estimated increase in the number of target molecules of 1x10⁺⁵ to 1x10⁺⁶.

### B. Solution Sandwich Hybridization

### 1) Denaturation:

10 µl of amplified target DNA from each of the above aliquots was combined with 30 µl of H₂O in a 1.5-ml Eppendorf tube and placed in a boiling water bath for 10 minutes. The tubes were then transferred to a chilled water bath (4°C) for two minutes and then centrifuged in a microcentrifuge for 10 seconds.

### 2) Hybridization:

The denatured samples were split into two 20-µl portions (in Eppendorf tubes) in order that duplicate samples could be run. 100 µl of hybridization mix that had been pre-equilibrated at 37°C for 10 minutes was added. Hybridization mix (HM) was prepared by combining 1987 µl of hybridization buffer, 12.4 µl of alkaline phosphatase-labeled reporter probe (6.2 x 10⁻¹² moles), 1.5 µl of biotinylated capture probe (4.5 x 10⁻¹¹ moles), and 40 µl of a 50 mg/ml solution of bovine serum albumin (BSA). The sequences of the probes are shown below. Hybridization buffer (HB) was prepared by combining: 3 ml of 20X SSC, pH 7.0, 0.1 ml of Triton® X-100, an alkylaryl polyether alcohol having 9-10 ethoxy units, 1.0 ml of deionized formamide, 6.375 ml of H₂O, and 25 µl of 1.0 N HCl. The samples were hybridized for 10 minutes at 37°C.

5'-CCCAGTATTTGTCTACAGCCTTCTG
Reporter Probe
5'-CAGGCCAGGATTAACTGCGAATCGT
Capture Probe

### C. Immobilization and Detection

Hybridized sample solutions prepared as described above were immobilized using three different solid supports: streptavidin-coated chromium dioxide particles, streptavidin coated microtiter plates, and streptavidin coated membranes as described above.

### 1) Immobilization and Detection on Streptavidin Coated Chromium Dioxide Particles:

120 µl aliquots of solution sandwich hybridization mixtures of each target nucleic acid amplification dilution levels were added to 12 µl of streptavidin-coated chromium dioxide particles (12 µg), prepared as described in U.S. 4,661,408). The samples were then incubated for 10 minutes at 37°C, centrifuged in a microfuge for 5 seconds, and placed in a Corning magnetic rack for two minutes at 25°C. The pellets were washed twice at 25°C by adding 200 µl of wash buffer (WB) containing 1X SSC, pH 7.0 and 0.17% Triton® X-100, mixing, placing the samples in a magnetic rack for 2 minutes, and removing the wash buffer. The samples were washed a third time as described above except that they were incubated for 5 minutes at 37°C prior to placing them in the magnetic rack.

Detection was accomplished by adding 50 µl of an alkaline phosphatase substrate solution to each sample containing 1 M diethanolamine, pH 8.9, 5 mM MgCl₂, 2 mM zinc acetate, 2 mM N-(2-hydroxyethyl)ethylene-diaminetriacetic acid (HEDTA), and 200 µM 4-methylumbelliferyl phosphate and 200 µM p-nitrophenyl phosphate, for fluorescence and chromogenic detection, respectively. The samples were incubated for 2 hours at 37°C. For fluorescence detection, 10 µl of each sample was diluted with 390 µl of water. The fluorescence signal generated for each sample was measured in a SPEX F212 spectrofluorometer by exciting at 365nm and measuring the emitted fluorescence at 450nm. For chromogenic detection, the presence of p-nitrophenol was detected by measuring the absorbance of the samples at 405nm.

### 2) Immobilization and Detection on Streptavidin Coated Microtiter Plates

Streptavidin was coated onto microtiter plates as follows: 100 µl of a 10 µg/ml solution of streptavidin prepared in a 0.1 M sodium carbonate buffer, pH 9.6, was added to each well of a micro-titer plate and allowed to bind overnight (16 hours) at 4°C. The wells were then washed three times at 25°C in a wash buffer which was 15 mM in sodium citrate, 150 mM in sodium chloride, and contained 0.17% Triton® X-100. The wells were then blocked by adding 300 µl of 1X phosphate buffered saline (PBS), pH 7.4, containing 2% bovine serum albumin (BSA) and 0.01% thimerosal, and incubated for 2 hours at 37°C. The microtiter plates were stored in this buffer at 4°C until needed, at which time they were washed once at 25°C in wash buffer.

100 µl of the solution sandwich hybridization mixture for each target nucleic amplification level was added to individual wells in the microtiter plate and incubated at 37°C for one hour. Each well was then washed three times with wash buffer at 25°C. 100 µl of substrate buffer (as described above) containing either 4-methylumbelliferyl phosphate or p-nitrophenyl phosphate, was added to each well of the microtiter plate. The plate was incubated at 37°C. Fluorescence due to the formation of 4-methylumbelliferone was measured as described above after 3 hours and the presence of p-nitrophenol was detected by measuring the absorbance of the samples at 405nm after 2.5 hours in a spectrophotometer.

### 3) Immobilization and Detection in a Membrane Device:

Membrane devices were prepared as described in applicants' assignee's copending application, see above. For the purpose of this assay, 50 µg of streptavidin in a 2 µl volume containing 1X PBS was spotted onto the permeable top membrane of the device. The membranes were dried at 37°C for 5 minutes. 500 µl of 5% fish gelatin in 1X PBS was then added to each membrane and incubated overnight at 37°C. The devices were then assembled as described in applicants' assignee's copending application, see above. For immobilization, 100 µl of the solution sandwich hybridization mixture for each target nucleic amplification level was diluted to 400 µl with Herptran™ and added to individual membrane devices, which were allowed to stand at 25°C for 10 minutes. Wash buffer (200 µl of 0.1 M Tris, pH 9.5, containing 0.05% Tween®20) was added and allowed to flow through the devices. 200 µl of substrate solution (0.1 M Tris, pH 9.6, containing BCIP/NBT as described in applicants' assignee's copending application) was then added to each device which were then incubated for 10 minutes at 25°C. Another 200 µl of substrate solution was then added to each sample which were allowed to incubate for another 10 minutes at 25°C. 200 µl of stop solution (1.0 N HCl) was added to each sample. The presence of colored spots in the center of the membranes was indicative of a positive response.

### EXAMPLE II

### Detection of HIV I DNA - Comparative Example

### A. Immobilization of Amplified Target DNA

The amplified target DNA from each of the aliquots in Example I was immobilized on GENE SCREEN™ hybridization membrane by first denaturing 5 µl of each dilution reaction in 95 µl of 0.2 N sodium hydroxide at room temperature for one min. and then filtering the reactions products through the membrane using a dot blot device. The DNA resulting from the PCR process is then retained on the GENE SCREEN surface. The wells of the dot blot device were then rinsed with 200 µl of 5X SSC. The membrane was then removed from the dot blot device and exposed to ultraviolet light at 302nm for five minutes, which linked the PCR products to the membrane surface.

Hybridization between the immobilized PCR products and the reporter probe utilized in Example I was then carried out in a sealable pouch. This was performed by first prehybridizing the membrane with the immobilized PCR products for 10 minutes at 50°C in 1 ml of a buffer containing 5X SSC, 0.5% bovine serum albumin, 0.5% polyvinylpyrrolidone, and 1% SDS, and then adding the alkaline phosphatase labeled reporter probe to the hybridization pouch at a final concentration of 2.5 nM. The membrane was hybridized for 15 minutes at 50°C. The membrane was removed from the sealable pouch and washed twice by agitating the membrane in a solution of 250 ml 1X SSC, containing 1% SDS for 5 minutes at 45°C in order to remove any unhybridized probe. The membrane was further washed twice by agitating the membrane in 250 mL of 1x SSC, containing 1% Triton® X-100, at 45°C for five minutes. Finally, the membrane was washed once agitating in 250 ml of 1X SSC at room temperature for 5 minutes.

### B. Detection

The hybridized probe was detected by placing the membrane in 8 mL of development buffer (10 mM Tris, pH 9.6, containing 50 mM NaCl, 10 mM MgCl₂, and 10 µm of each of NBT and BCIP). The reaction was incubated at 37°C for 30 minutes. Probe hybridized to the PCR reaction products was visualized on the membrane by the deposition of dye resulting from the alkaline phosphatase activity of the probe.

The results of this experiment and the data from Example I utilizing the assay of this invention are summarized in Table I. The use of the solution sandwich assay in conjunction with PCR, the assay of this invention, results in surprisingly greater sensitivity than does combining the PCR amplification method with the dot blot assay method. In the dot blot direct assay method, background of nonspecific origin obscured the detection below the 1000 copy level of pre-amplified pBH10-R3 target DNA.

**Table I**

| ASSAY SENSITIVITY | | | | |
|---|---|---|---|---|
| ASSAY TYPE | COPIES DETECTED | | PCR CYCLES | TIME |
| | CHROM. | FLUOR. | | |
| DIRECT - [DOT BLOT] | 1000 | - | 30 | 6 hr. |

| THIS INVENTION | | | | |
|---|---|---|---|---|
| · MAGNETIC PARTICLE | 1000 (50) | 100 (5) | 20 | 5 hr. |
| · MICROTITER PLATE | 1000 (50) | 100 (5) | 30 | 6 hr. |
| · MEMBRANE DEVICE | 100 (5) | - | 30 | 5 hr. |
| Note: the values in parenthesis indicate the approximate levels of sensitivity as indicated by the fact that only 5% of the amplification reaction volumes was used for each assay point in each assay format and immobilization/detection method. | | | | |

## Claims

1. A nucleic acid probe assay for the detection and/or measurement of a preselected nucleic acid sequence in a sample suspected of including a nucleic acid containing said preselected sequence comprises the steps of:
(A) rendering the target nucleic acid single-stranded;
(B) amplifying at least one specific nucleic acid sequence contained within the preselected nucleic acid sequence by
(1) treating the strands with two oligonucleotide primers, for each different specific sequence being amplified, under conditions such that for each different sequence being amplified an extension product of each primer is synthesized which is complementary to each nucleic acid strand, wherein said primers are selected so as to be sufficiently complementary to the different strands of each specific sequence to hybridize therewith such that the extension product synthesized from one primer, when it is separated from its complement, can serve as a template for synthesis of the extension product of the other primer;
(2) separating the primer extension products from the templates on which they were synthesized to produce single-stranded molecules; and
(3) treating the single-stranded molecules generated from step (2) with the primers of step (1) under conditions that a primer extension product is synthesized using each of the single strands produced in step (2) as a template;
(4) repeating steps (2) and (3) to produce sufficient primer extension product for detection and/or measurement:
(C) rendering the product of step (B)(4) single-stranded;
(D) contacting the product of step (C) with capture and reporter probes in solution wherein said capture probe is complementary to a portion of a primer extension product not including the nucleic acid sequences defined by both primers; wherein said reporter probe is complementary to a portion of a primer extension product not including the nucleic acid sequences defined by the capture probe and by both primers; and wherein the capture and reporter probes are complementary to the same nucleic acid strand;
(E) immobilizing the sandwich product of step (D) by contacting it with a solid support having an anchor receptor group on its surface capable of interacting with the anchor group of the capture probe;
(F) removing any unhybridized reporter probe; and
(G) detecting and/or measuring the reporter group immobilized on the solid support.

2. The assay of claim 1 wherein the solid support is a selectively permeable membrane contained within a device said device having multiple layers comprising: (a) a permeable membrane having a capture reagent attached thereto, (b) an intermediate layer, and (c) an absorbent layer, wherein layer (b) is in direct communication with layers (a) and (c), the improvement wherein layer (b) is a selectively permeable membrane and has at least one hole therethrough, the hole is directly below the capture reagent, and the area of said hole or the combined area of a plurality of holes is less than the area covered by the capture reagent.

3. The assay of claim 1 wherein said preselected nucleic acid sequence is HIV I DNA.

## Patentansprüche

1. Nucleinsäuresonden-Assay für den Nachweis und/oder die Messung einer im voraus gewählten Nucleinsäuresequenz in einer Probe, von der man annimmt, daß sie eine Nucleinsäure umfaßt, die diese im voraus gewählte Sequenz enthält, umfassend die Schritte:
(A) Überführen der Zielnucleinsäure in den einzelsträngigen Zustand;
(B) Amplifizieren wenigstens einer spezifischen Nucleinsäuresequenz, die in der im voraus gewählten Nucleinsäuresequenz enthalten ist, durch
(1) Behandeln der Stränge mit zwei OligonucleotidPrimern für jede unterschiedliche zu amplifizierende spezifische Sequenz unter solchen Bedingungen, daß für jede unterschiedliche zu amplifizierende Sequenz ein Verlängerungsprodukt jedes Primers synthetisiert wird, das komplementär zu jedem Nucleinsäurestrang ist, wobei die Primer so ausgewählt werden, daß sie zu den unterschiedlichen Strängen jeder spezifischen Sequenz hinreichend komplementär sind, um damit so zu hybridisieren, daß das aus einem Primer synthetisierte Verlängerungsprodukt, wenn es von seinem Komplement getrennt wird, als Matrize für die Synthese des Verlängerungsprodukts des anderen Primers dienen kann;
(2) Trennen der Primer-Verlängerungsprodukte von den Matrizen, an denen sie synthetisiert wurden unter Bildung einzelsträngiger Moleküle; sowie
(3) Behandeln der in Schritt (2) erzeugten einzelsträngigen Moleküle mit den Primern von Schritt (1) unter Bedingungen, daß ein Primer-Verlängerungsprodukt unter Verwendung jedes der in Schritt (2) erzeugten Einzelstränge als Matrize synthetisiert wird;
(4) Wiederholen der Schritte (2) und (3), um ausreichend Primer-Verlängerungsprodukt für einen Nachweis und/oder eine Messung zu erzeugen;
(C) Überführen des Produkts aus Schritt (B) (4) in den einzelsträngigen Zustand;
(D) In-Kontakt-Bringen des Produkts aus Schritt (C) mit Abfang- und Reportersonden in Lösung, wobei die Abfangsonde komplementär zu einem Teil eines Primer-Verlängerungsprodukts ist, das die durch beide Primer definierten Nucleinsäuresequenzen nicht enthält, wobei die Reportersonde komplementär zu einem Teil eines Primer-Verlängerungsprodukts ist, das die durch die Abfangsonde und die beiden Primer definierten Nucleinsäuresequenzen nicht enthält, und wobei die Abfang- und die Reportersonde komplementär zu demselben Nucleinsäurestrang sind;
(E) Immobilisieren des Sandwich-Produkts aus Schritt (D), indem man es mit einem festen Träger in Kontakt bringt, der eine Anker-Rezeptorgruppe auf seiner Oberfläche aufweist, die mit der Ankergruppe der Abfangsonde wechselwirken kann;
(F) Entfernen jeder unhybridisierten Reportersonde; und
(G) Nachweisen und/oder Messen der auf dem festen Träger immobilisierten Reportergruppe.

2. Assay gemäß Anspruch 1, wobei der feste Träger eine selektiv durchlässige Membran ist, die in einer Vorrichtung enthalten ist, wobei die Vorrichtung über mehrere Schichten verfügt, umfassend: (a) eine durchlässige Membran mit einem daran befestigten Abfangreagenz, (b) eine Zwischenschicht und (c) eine Absorptionsschicht, wobei Schicht (b) in direkter Verbindung mit den Schichten (a) und (c) steht, wobei Schicht (b) eine selektiv durchlässige Membran ist und wenigstens ein Loch durch sie aufweist, das Loch sich direkt unterhalb des Abfangreagenzes befindet und die Fläche des Loches oder die kombinierte Fläche mehrerer Löcher geringer ist als die vom Abfangreagenz bedeckte Fläche.

3. Assay gemäß Anspruch 1, wobei die im voraus gewählte Nucleinsäuresequenz HIV-I-DNA ist.

## Revendications

1. Un test utilisant des sondes nucléotidiques pour la détection et/ou la quantification d'une séquence d'acide nucléique présélectionnée dans un échantillon dans lequel on suspecte la présence d'un acide nucléique contenant ladite séquence présélectionnée, qui comprend les étapes de:
(A) mise sous forme simple brin de l'acide nucléique cible;
(B) amplification d'au moins une séquence nuclèotidique spécifique présente dans la séquence nucléotidique présélectionnée par:
(1) traitement des brins avec deux amorces oligonucléotidiques, pour chaque séquence spécifique différente devant être amplifiée, dans des conditions telles que pour chaque séquence différente devant être amplifiée, un produit d'extension de chaque amorce est synthétisé, qui est complémentaire à chaque brin d'acide nucléique, dans lequel lesdites amorces sont sélectionnées de façon à être suffisamment complémentaires aux brins différents de chaque séquence spécifique pour s'hybrider avec ces dernières de sorte que le produit d'extension synthétisé à partir d'une amorce, lorsqu'il est séparé de son brin complémentaire, puisse être utilisé en tant que matrice pour la synthèse du produit d'extension de l'autre amorce;
(2) séparation des produits d'extension d'amorce à partir des matrices sur lesquelles ils sont synthétisés pour produire des molécules simple brin; et
(3) traitement des molécules simple brin générées dans l'étape (2) avec les amorces de l'étape (1) dans des conditions telles que un produit d'extension d'amorce soit synthétisé en utilisant chacun des simples brins produits dans l'étape 2 en tant que matrice;
(4) répétition des étapes (2) et (3) pour produire une quantité suffisante de produit d'extension d'amorce pour permettre une détection et/ou une quantification;
(C) mise sous forme simple brin du produit de l'étape (B)(4);
(D) mise en contact du produit de l'étape (C) avec des sondes de capture et de détection en solution dans laquelle ladite sonde de capture est complémentaire à une partie du produit d'extension d'amorce n'incluant pas les séquences nucléotidiques définies par les deux amorces; dans laquelle ladite sonde de détection est complémentaire à une partie du produit d'extension d'amorce n'incluant pas les séquences nucléotidiques définies par la sonde de capture et par les deux amorces; et dans laquelle les sondes de capture et de détection sont complémentaires au même brin d'acide nucléique;
(E) l'immohilisation du produit en sandwich de l'étape (D) par mise en contact de ce dernier avec un support solide présentant un groupe récepteur d'ancrage sur sa surface capable d'interagir avec le groupe d'ancrage de la sonde de capture;
(F) élimination de toute sonde de détection nonhybridée; et
(G) détection et/ou quantification du groupe de détection immobilisé sur le support solide.

2. Le test selon la revendication 1 dans lequel le support solide correspond à une membrane de perméabilité sélective contenue dans un dispositif, ledit dispositif comportant des couches multiples comprenant:
(a) une membrane imperméable à laquelle est liée un réactif de capture;
(b) une couche intermédiaire; et
(c) une couche absorbante, la couche (b) étant en communication directe avec les couches (a) et (c),
caractérisé en ce que la couche (b) correspond à une membrane de perméabilité sélective et elle est traversée par au moins un trou, le trou étant directement en-dessous du réactif de capture, et la surface dudit trou ou la surface combinée d'une pluralité de trous est inférieure à la surface couverte par le réactif de capture.

3. Le test selon la revendication 1, dans lequel ladite séquence nucléotidique présélectionnée correspond à un ADN d'HIV I.
